(19) Europäisches Patentamt
European Patent Office
Office européen des brevets

(11) **EP 2 749 319 A1**

(12) **EUROPEAN PATENT APPLICATION**
published in accordance with Art. 153(4) EPC

(43) Date of publication:
**02.07.2014 Bulletin 2014/27**

(21) Application number: **11773984.7**

(22) Date of filing: **31.08.2011**

(51) Int Cl.:
*A61Q 1/04* (2006.01)   *A61Q 1/06* (2006.01)
*A61K 8/25* (2006.01)   *A61K 8/02* (2006.01)
*A61K 8/68* (2006.01)

(86) International application number:
**PCT/BR2011/000306**

(87) International publication number:
**WO 2013/029125 (07.03.2013 Gazette 2013/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(71) Applicant: **Natura Cosméticos S.A.
06882-700 Itapecerica da Serra SP (BR)**

(72) Inventors:
• **NAGAMATSU, Ana Paula
12322-230 - São Paulo - SP (BR)**

• **WADA, Joyce Kazue Alves
13207- 345 - Jundiaí - SP (BR)**
• **SPINA, Marcos Rogerio
13209-460 - Jundiaí- Sp (BR)**

(74) Representative: **Pearson, Samuel John et al
Abel & Imray
20 Red Lion Street
London WC1R 4PQ (GB)**

(54) **COSMETIC COMPOSITION FOR THE LIPS CONTAINING SPHERICAL SILICA MICROPARTICLES**

(57) The present invention discloses a cosmetic for the lips comprising spherical micro particles of silica, emollients and gel, as well as cosmetically acceptable excipients.

The present invention also refers to the fabrication process of the said cosmetic and the use thereof.

Fig. 1

**Description**

FIELD OF THE INVENTION

The present invention relates to cosmetic compositions for the lips. More specifically, lipsticks and lip glosses containing spherical micro particles, emollients and film formers are disclosed.

BACKGROUND OF THE INVENTION

Lipstick is a cosmetic product which allows, preferably, the enhancement of the lips through coloring. There are several techniques for the production of lipsticks with variants relating to the texture and colors.

When a lipstick is shiny, it is called lip gloss.

The habit of coloring the lips comes from ancient Egypt, more than 4000 years ago; however, the solid formula for lipstick appeared in the 1930's. Even so, most basic formulations for lipsticks (which consist of a waxy base) has not suffered radical changes. Up to now, it is a dispersion of colors in a fat base, permitting the easy application of a uniform layer.

The constant search for technologies for the development of lipsticks which exceed customer's expectations has been a challenge to researchers.

For the development of more effective products, a great advancement of cosmetic science has been necessary, as well as the use of ever more advanced techniques.

For the development of good products, quantitative and qualitative techniques are necessary for the characterization and knowledge of the effects, and the subsequent characterization of the products for each type of application.

According to the US Patent 6,548,075, although several cosmetic products for innumerable purposes are known, there is a constant necessity for new products which present improvements. Particularly, the most desirable products produce a pleasant sensation as they are applied to the skin, transmitting a smooth and soft sensation. The invention of said patent provides a topical preparation with spherical micro particles which consist totally or partially of at least one linear polyglycan which is not soluble in water. The addition of these micro particles increases the sensation of softness on the skin due to the regular shape of the micro particles, which perform a rolling effect.

Patent application WO 2009022306 deals with dyes in the cosmetic industry. Organic pigments are widely used; however, the use of these pigments is limited due to security problems and pharmacotechnical problems. A solution to this problem is the incorporation of these pigments in particles, preferably in the spherical shape, of an encapsulating agent containing silica. The particles are preferably spherical to confer a lubricant rolling effect.

As disclosed in Patent EP 1 439 816, the use of spherical particles of silicon dioxide in cosmetic preparations is known. These particles are used in decorative cosmetics to produce a "soft" effect due to the distribution of light. The present invention uses particles of silica having a size of 3-15 $\mu$m and a pore volume of 0.2-0.4 mL/g. The particle size, as well as the pore volume used in the present invention are, therefore, smaller than those found in the state of the art, conferring a superior filling effect of the labial cavities, which provides softness and an improved rolling effect in relation to those described in the Patent EP 1 439 816.

The international publication WO2008022836 discloses the use of fumed silica in pharmaceutical preparations. This document discloses the advantages of the use of fumed silica in relation to silica gel, particularly as a result of its spherical nature. Further, in cosmetic compositions, the fumed silica can serve to improve the sensation of the application of the product on the skin, due to the rolling effect. The present invention uses particles of silica having a size of 3-15 $\mu$m and a pore volume of 0.2-0.4 mL/g. The particle size, as well as the pore volume used in the present invention are, therefore, smaller than those found in the state of the art, conferring a superior filling effect of the labial cavities, which provides smoothness and an improved rolling effect in relation to those described in the international publication WO 2008022836.

US Patent 5,034,216 discloses cosmetic compositions containing gel, spherical silica particles, which can have dyes and oils. The silica spheres have an average size of less than 15 microns, preferably 2-5 microns. The silica microspheres help give a silky effect to the final product. Further, the smoothness is increased by the silica treatment with a coating made of polyethylene or another polymer. This reference uses coated silica to increase smoothness, which differs from the present invention, which attains the improved rolling effect through the combination of other ingredients.

SUMMARY OF THE INVENTION

The present invention refers to lipsticks and lip glosses containing a combination of spherical micro particles, emollients and film formers, as well as other optional ingredients.

The spherical micro particles are composed of silicon dioxide (silica). Examples of micro particles which are useable in the present invention can be selected from micro particles currently commercially available from the company Presperse Inc., such as Spheron N-2000 (solid silica; average particle size: 2-15 micrometers) and Spheron P-1500 (porous silica; average particle size: 3-15 micrometers), preferably Spheron P-1500.

The emollients can be selected from the group comprising oil from castor oil plant seeds (ricinus communis), isocetyl stearoyl stearate, dicaprylyl ether, diisostearyl maleate, Octyldodecanol.

The film formers can be selected, for example, among polyacyladipate-2 of bis-diglyceryl and polyhydroxystearate/tetrabehenate of dipentaerithrytyl.

As examples of optional ingredients for the composition of the present invention passion fruit ceramide (helps in the restoration of the lips), cocoa/cupuaçu butter (moisturizer) and antioxidant complex (lycopene + Vitamin E + green coffee extract), pigments, sunscreens, ingredients with a high refractive index and structuring agents can be cited.

The technical problem solved by the present invention intends to resolve is to provide a lipstick and/or lip gloss with sliding and delivery of high coverage in a single application, supplying the renewal and hydration of the lips (such sensation can be felt even after the removal of the product from the lips), these two effects acting in synergy. The synergetic effect was perceived through sensorial research with consumers in comparison with other products on the market. Therefore, there are no available tests to verify this result. The effect provided by the spherical particles eases the delivery of the actives which renew and hydrate the lips.

The performance of the final product of the present invention was compared with products of the state of the art, as well as with a control, as described in details in the detailed description of the invention.

BRIEF DESCRIPTION OF THE FIGURES

Figure 1: market research test - importance of the characteristics (%).
Figure 2: market research test - use of the products in the test (%).
Figure 3: market research test - general evaluation (%).
Figure 4: market research test - global appearance of the stick before application (%).
Figure 5: market research test - general evaluation of the color (%).
Figure 6: market research test - intensity of the color (%).
Figure 7: market research test - pleasantness of the aroma (%).
Figure 8: market research test - intensity of the fragrance (%).
Figure 9: market research test - pleasantness of the flavor (%).
Figure 10: market research test - ease of application (%).
Figure 11: market research test - sliding (%).
Figure 12: market research test - stick texture (%).
Figure 13: market research test - emollience (%).
Figure 14: market research test - amount discharged (%).
Figure 15: market research test - coverage (%).
Figure 16: market research test - thickness of the film on the lips (%).
Figure 17: market research test - creaminess of the film (%).
Figure 18: market research test - shine (%).
Figure 19: market research test - runniness (%).
Figure 20: market research test - hydration / softness (%).
Figure 21: market research test - duration time (%).
Figure 22: market research test - satisfaction with the duration (%).
Figure 23: market research test - fixation (%).
Figure 24: market research test - transfer (%).
Figure 25: market research test - innovation (%).
Figure 26: market research test - comparison with the usual (%).
Figure 27: market research test - preference.
Figure 28: market research test - adverse reactions or sensations of discomfort.
Figure 29: market research test - yes/no - sensation of hydrated / soft lips even without the lipstick (%).
Figure 30: market research test - yes/no - delivery of high coverage on the first swipe (%).
Figure 31: market research test - yes/no - sensation of protected lips (%).
Figure 32: market research test - yes/no - sensation of less dryness of the lips (%). S
Figure 33: market research test - yes/no - sensation that the volume of the lips increased (%).
Figure 34: market research test - yes/no - sensation that the dryness of the lips diminished, whitish skin peeling (%).
Figure 35: market research test - yes/no - pleasurable sensation in the mouth (%).

Figure 36: market research test - yes/no - sensation of hydrated lips for 24h (%).
Figure 37: market research test - yes/no - improvement in the definition of the lips (%).
Figure 38: market research test - yes/no - sensation of improvement of the general appearance of the lips (%).
Figure 39: market research test - yes/no - sensation of comfort (%).
Figure 40: market research test - yes/no - does not leave the lips slimy / sticky (%).
Figure 41: market research test - yes/no - provides intense color in the first application (%).
Figure 42: market research test - yes/no - sensation that it hides the thin lines of the lips (%).
Figure 43: market research test - yes/no - sensation of softer lips with the use of the lipstick (%).
Figure 44: damage of the samples of the product of the present invention vs. control (%).
Figure 45: labial restoration conferred by the product of the present invention and control over time.
Figure 46: Values of hydration (ua) in relation to control for A1650/A1550.
Figure 47: hydration in relation to control for A1650/A1550 (%).

DETAILED DESCRIPTION OF THE INVENTION

The present invention provides cosmetics for use on the lips, particularly a lipstick and lip gloss, containing a combination of ingredients in the form of gel with emollients of different viscosities and spherical particles.
The spherical particles consist of silica microspheres, more preferably spherical particles sold as Silica Spheron® P-1500 and are used preferably in a concentration of 0,5 to 5% of the total by mass of the cosmetic composition. More preferably, the silica microspheres are present in about 0.5 - 2.5%.
The preferred emollients used in the present cosmetic composition are selected from cocoa butter, Bis-2-Digliceryl Polyacyladipate, vegetable lanolin, Octyldodecanol, among others. Preferably the emollients consist of 0.5 to 20% of the total by weight of the cosmetic composition.
Further, the cosmetic composition of the present invention can include dyes, flavorings, which contribute approximately 40% by weight of the cosmetic composition.
Particularly, the cosmetic composition of the present invention can comprise at least one of the components selected from the group consisting of: ceramides, cocoa butter, vegetable lanolin, Bis-diglycerylpolyacyladipate-2, lycopene, Vitamin E, green coffee extract, pigments, film forming agents, sunscreens, ingredients with a high refractive index and mixtures thereof.
For the execution of the composition of the invention in the form of a lipstick a waxy base is used.
Yet more preferably, the base for the lipstick of the invention can comprise:

| Component name | Concentration (%) |
|---|---|
| Isocetyl stearoyl stearate | 5,0 - 25,0 |
| Diisostearyl malate | 1,0 - 5,0 |
| Dicaprylyl ether | 4,0 - 19,0 |
| Glyceryl abietate | 0,5 - 3,0 |
| Castor oil | 0,5 - 25,0 |
| Fractionated-hydrogenated vegetable fat | 0,3 - 3,0 |
| Lycopene | 0,02 - 0,15 |
| Ceresin p | 1,0 - 4,0 |
| Microcrystalline wax | 1,0 - 4,0 |
| Bis-diglyceryl 2-polyacyladipate | 0,5 - 3,0 |
| Dipentaeritrityl polyhydroxystearate | 0,5 - 3,0 |
| Candellila wax | 1,0 - 10,0 |
| Organic carnauba palm wax | 1,0 - 10,0 |

In a particularly preferred embodiment of the invention, the cosmetic compositions comprise:

| Component name | Concentration (%) |
|---|---|
| Lipstick base (according to the present invention) | 40,0 - 60,0 |
| Maleated castor oil | 0,5 - 2,5 |
| Theobrom cacao seed butter | 0,1 - 3 |
| Octyldodecanol | 1,0 - 10,0 |
| Dyes | 2,0 - 20,0 |
| Fractionated-hydrogenated vegetable fat | 0,3 - 3,0 |
| Castor oil | 0,5 - 25,0 |
| Dicaprylyl ether | 4,0 - 19,0 |
| Silica microspheres | 0,5 - 2,5 |
| 3-[2-(ethylhexyl)oxyl]-1,2-propanediol | 0,2 - 0,7 |
| Flavoring | 0,5 - 2,5 |
| Passion fruit ceramides | 0,2 - 1,0 |

The micro particles present a parallel phase of the process, that is, they are pre-dispersed with emollient and added after the incorporation of the product's coloring. This process guarantees the correct structuring of the formula in the production process.

The lipstick and lip gloss of the present invention provide healthy and renewed lips, preventing and improving lip dryness. This benefit is due to the following components: passion fruit ceramides, cocoa butter and, optionally, Bis-2-Digliceril Polyacyladipate.

Through the deposition of the molecules of ceramides on the skin of the lips, the fortification and hydration of the labial barrier is achieved. The cocoa butter promotes softness and hydration in the lips, avoiding dryness in them (emollience). The Bis-2-Digliceril Polyacyladipate, which is a vegetable origin ingredient ("lanoline like") with a high hydration power, absorbs water, maintaining it on the labial surface. Further, the optional use of carnauba palm wax confers the formation of a film. The carnauba palm wax, together with gel agents, forms a creamy film over the skin, avoiding the loss of water. This result was demonstrated in the labial restoration test, described below.

Another effect provided by the present lipstick / lip gloss is the promotion of a more uniform texture of the lips. This is due to the ingredients: passion fruit ceramides and cocoa butter. The effect of the ceramides is achieved as above, and the cocoa butter, through the promotion of the softness and hydration of the lips, avoids dryness of the lips and consequent peeling, maintaining the uniformity of the skin surface. Optionally, one can use vegetable lanolin, which has a high capacity to absorb water, retaining it for longer on the skin. Promotes softness and hydration of the lips, avoiding dryness, making them more healthy and uniform. This result was demonstrated in the labial restoration test, described below.

The use of the presently disclosed lipstick improves the appearance of the lips with the smoothening of "fine lines". This effect is a result of the formulation in question.

The deposition of the ceramide molecules over the skin of the lips promotes the uniformity of the surface of the lips and, therefore, results in the minimization of the appearance of thin lines, while the cocoa butter promotes softness and hydration of the lips, avoiding dryness and consequently peeling, avoiding cracked lips. With the increase of the hydration of the lips the tumescence of the cells occurs, which can minimize the appearance of thin lines. Further, the use of transparent waxes and emollients with a high refractive index confers high shininess upon the application of the product over the lips, and through an optical effect, disguises the thin lines. Finally, highly reflective sparklers, through an optical effect, also disguise the referred-to thin lines. The use of emollients, film forming agents and a high concentration of ultra thin pigment enables the formation of a silky and comfortable film, which helps to prevent the loss of water. The retention of water in the lips promotes their tumescence, improving their appearance with the minimization of thin lines. Optionally, the vegetable lanolin promotes softness and hydration in the lips, avoids dryness and peeling. With the hydration of the skin, the cells become more tumesced minimizing the depth of the referred-to thinner lines. Further, ingredients with a high refractive index, through an optical effect, reflect the light, disguising the thinnest lines. This result was demonstrated in the hydration and labial restoration test, described below.

The formulation of the new lipstick promotes immediate hydration for up to 24 hours. This is also due to the components passion fruit ceramides, Bis-diglyceryl 2-polyacyladipate / vegetable lanolin, and film forming agents.

The deposition of ceramides molecules over the skin of the lips, promoting the fortification of the labial barrier, minimizes the loss of water, while the Bis-diglyceryl 2-polyacyladipate, a vegetable origin ingredient ("lanoline like") with a high hydration power, absorbs water, maintaining it over the labial surface. Further, the hydration occurs due to the exclusive combination of carnauba palm wax with gel agents which deposit themselves over the skin forming a comfortable film which prevents the loss of water. This result was demonstrated in the hydration test, described below.

The lipstick of the present invention promotes the restoration of the lips after 1 hour of proper use, mainly due to the effect of the passion fruit ceramides, cocoa butter and Bis-diglyceryl 2-polyacyladipate, as disclosed above. This result was disclosed in the labial restoration test, described below.

Further, due to the singular characteristics of the present formulation, a visual effect of the increase of the volume of the lips is obtained. This optical effect is obtained through the transparent waxes and emollients with a high refractive index, associated to ultrathin pigments, which provide incomparable shine and color effects and high performance and give the impression of an increase of the volume of the lips. This effect was evaluated according to the market research test, described further below.

Further, optionally, lycopene, vitamin E and green coffee extract can be included into the formulation. These components aid in the prevention of signs of aging and in protecting against free radicals.

Through the use of the presently disclosed lipstick, one can obtain more vibrant lips, especially due to the optical effect caused by the high refractive index of the formula. Furthermore, a smooth coverage that surrounds the lips can be observed (a visual effect due to the low coverage of pigments). This result was also demonstrated in the market research test, described ahead.

In using the lipstick, a smooth application that "melts"/ "hugs" the lips can be verified. This is due to the combination of ingredients in the form of a gel with emollients of different viscosities and spherical particles. The heat of the lips associated to the force necessary to slide the lipstick, makes the spherical particles slide over the lips, emitting the emollients. As the emollients are of different viscosities and are in the form of a gel, one has the sensation that the lipstick is "melting"/"hugging" the lips. This result was demonstrated in the market research test, described ahead.

Also a low coverage is obtained, highlighting the naturalness of the lips. This is attained through the combination of the dispersion of pigments and emollients. The low concentration of pigments associated with emollients with a high refractive index gives transparency to the application. This result was demonstrated in the market research test, described ahead.

Also a high sliding is obtained with maximum comfort and a smooth application which leaves the lips soft, both through the combination of emollients, gel and spherical particles. Due to the form of the spherical particles and their property of absorbing the emollients, in pressing the product over the lips, high sliding occurs with the emission of the emollients which leave a soft, smooth and brilliant film on the lips. The emollients in gel provide the creamy and comfortable sensation. This result was demonstrated in the market research test, described ahead.

The delivery of high coverage is also attained right from the first application due to spherical particles combined with emollients with different viscosities and in the form of a gel. The spherical particles present in the formulation act with a rolling effect, providing the deposition of the color in the first application. Besides the physical effect, these particles become tumesced with emollients and, when they are pressed, emit the emollients and ease the deposition of the pigments over the lips. The high coverage also is due to the concentration of pigments. This effect was tested according to the market research test, described further below.

A sensation of soft and hydrated lips is obtained, even without the lipstick due to the film forming agents. The lipstick deposits over the lips a comfortable film which protects and hydrates. The combination of film forming agents with emollients in gel and the spherical particles leaves a film over the lips giving the impression that the lips are soft/hydrated even without the lipstick. This effect was tested according to the market research test, described further below.

As well as a creamy texture which leaves the lips soft and hydrated, and protected, optionally the lipstick of the present invention can contain sunscreens, which protect the lips from UVB and UVA rays. This result was demonstrated in the market research test, described ahead.

A 3D shine can also be obtained through the optical effect of the transparent waxes and emollients with a high refractive index associated to specific sparklers.

With respect to the pharmacotechnical of the present lipstick, a stick in a silicone mold with a different crystallization was obtained, which conferred greater softness, creaminess and shine to the stick.

Tests carried out

The product of the present invention, tested as disclosed below, presents the following formulation:

Table 1: composition of the cosmetic base for lips of the present invention

| Component name | Concentration (%) |
|---|---|
| Isocetyl stearoyl stearate[1] | 26,9300 |
| Diisostearyl malate[3] | 6,0000 |
| Dicaprylyl ether[3] | 26,0000 |
| Glyceryl abietate[2] | 3,0000 |
| Castor oil[3] | 2,3000 |
| Fractionated-hydrogenated vegetable fat[3] | 1,0000 |
| Lycopene[4] | 0,0700 |
| Ceresin p[3] | 5,0000 |
| Microcrystalline wax[3] | 3,0000 |
| Bis-diglyceryl 2-polyacyladipate[4] | 3,0000 |
| Dipentaerythrityl polyhydroxystearate[4] | 3,70000 |
| Candellila wax[3] | 12,0000 |
| Organic carnauba palm wax[3] | 8,0000 |
| 1 - emollient; 2 - film former; 3 - structuring; 4 - anti-oxidant. | |

Table 2: composition of the cosmetic for lips of the present invention

| Component name | Concentration (%) |
|---|---|
| Base (according to Table 1 above) | 46,8960 |
| Maleated castor oil | 1,0000 |
| Theobrom cacao seed butter | 1,0000 |
| Octyldodecanol | 3,0000 |
| Dyes | 38,2040 |
| Fractionated-hydrogenated vegetable fat | 1,4000 |
| Castor oil | 0,6000 |
| Dicaprylyl ether | 3,0000 |
| Silica microspheres | 2,0000 |
| 3-[2-(ethylhexyl)oxyl]-1,2-propanediol[5] | 0,5000 |
| Flavoring | 2,0000 |
| Passion fruit ceramides | 0,4000 |
| 5 - preservative | |

- *Test 1: Market research test*

The objective of this test is the selection of the prototype with the best sensory performance through an affective quantitative evaluation of the domestic use of products with a personal evaluation interview. Five samples were randomly used by the participants (D7R, S2R, G5L, X4T and M8Y) during seven consecutive days. The samples X4T, M8Y and G5L are samples of the product of the present invention. The sample D7R is a sample of a competitor without spherical particles and the sample S2R is a sample of a competitor containing spherical particles, however with different characteristics to those of the present invention.

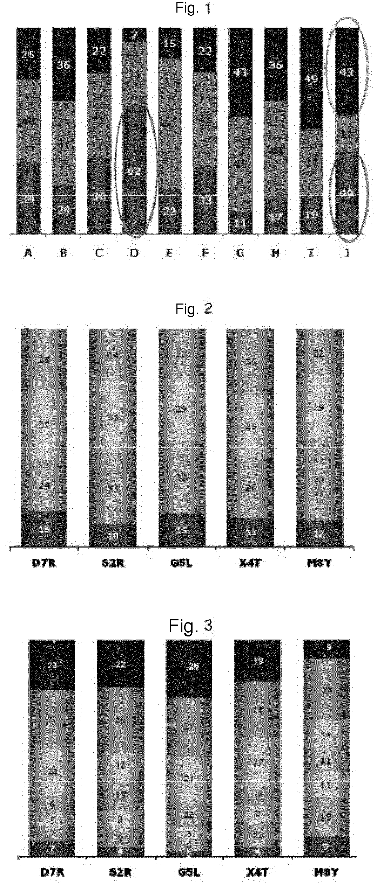

According to Figure 1, characteristics considered to be very important in a lipstick are the capacity to leave a creamy, but thin film on the lips; promote the restoration of the lips and smooth the lines of the lips. The superior part of the bars corresponds to the percentage of "very important", while the central part of the bars corresponds to the percentage of "moderate importance" and the inferior part corresponds to the percentage considered of "little importance". The letter A corresponds to the characteristic "present high sliding at the moment of application"; B corresponds to "provide high coverage in the 1st application, that is, the 1st swipe; C corresponds to "prevention of aging"; D corresponds to "have FPS 15 and UVA protection"; AND corresponds to "provider softness to the lips "; F corresponds to "have a long duration", G corresponds to "leave a creamy, yet thin film on the lips", H corresponds to "promote the restoration of the lips", I corresponds to "smooth the lines on the lips ": J corresponds to "having a color which I like".

A Figure 2 demonstrates the percentage of times used by the participants of the test of each lipstick tested. The superior part of the bars corresponds to the percentage of "more than three times a day", while the part just below corresponds to the percentage of "three times a day", the next to last part corresponds to the percentage of "two times a day" and the last part (inferior) of the bars corresponds to the percentage "once a day". As can be observed, the minority of the interviewees used the products only once a day. The same pattern was observed for all of the samples.

As a general evaluation (see Figure 3), the sample G5L obtained the greatest average of acceptance, while the M8Y presented the worst performance in this respect. The type of the spherical micro particles of the samples M8Y and G5L is different in the formulations. M8Y does not use silica microspheres. With reference to Figures 3 to 5, the superior part of the bars corresponds to the percentage of "I like very much", while the part just below corresponds to the percentage of "I like moderately" and the part yet below corresponds to the percentage "I like slightly". The central part corresponds to the percentage "neither like, nor dislike" and the part just below to the percentage "I dislike slightly". The next to last part corresponds to the percentage of "I dislike moderately" and the last part (inferior) of the bars corresponds to the percentage "I dislike very much".

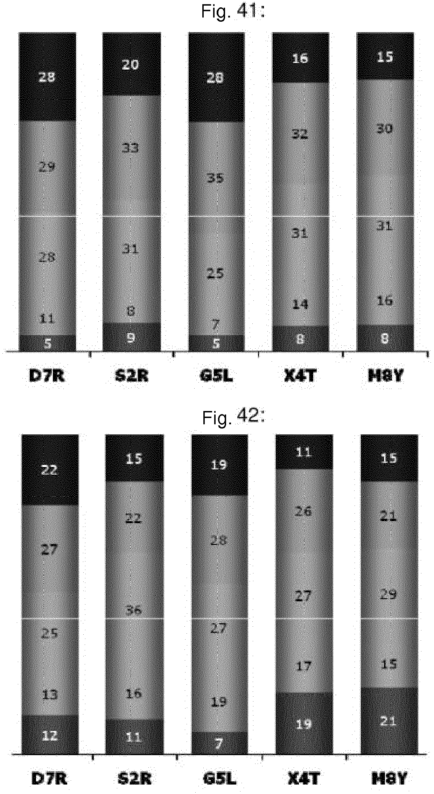

In respect to the global appearance of the stick before application (according to Figure 42) and the general evaluation of the color of the product, G5L presented the best general results, according to Figure 52.

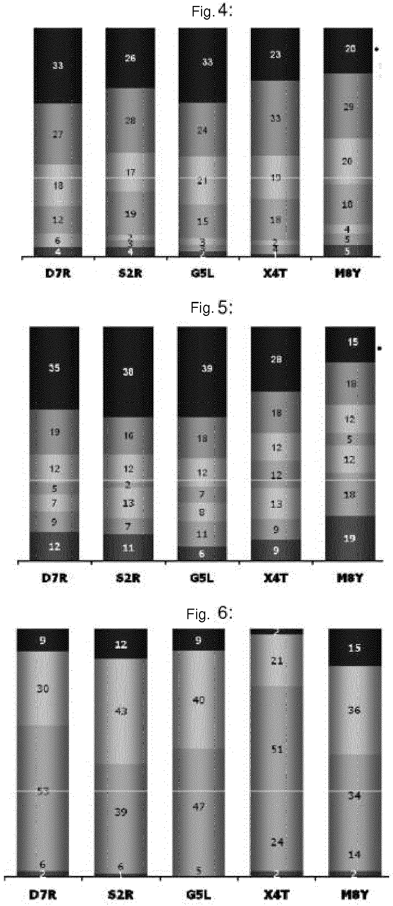

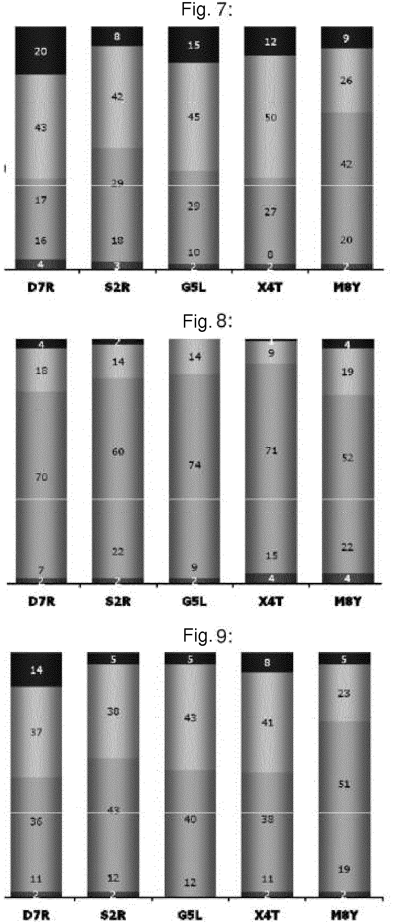

In relation to the intensity of the color, the samples D7R and X4T presented better rates of ideal intensity than S2R and M8Y, according to Figure 6. Reference is also made to Figures 6 and 8 where the superior part of the bars corresponds to the percentage of "A lot stronger than I like", while the part just below corresponds to the percentage of "Stronger than I like". The central part corresponds to the percentage "At the intensity that I like" and the part just below to the percentage "Weaker than I like". The last part (inferior) of the bars corresponds to the percentage "Much weaker than I like".

The samples with greatest percentage of "very pleasant" aroma were D7R and G5L (see Figure 7), while the intensity of the fragrance sample G5L was the closest to ideal (greatest percentage of "At the intensity that I like"), according to Figure 83. In this sense, in Figures 7 and 9 the superior part of the bars corresponds to the percentage of "Very pleasant", while the part just below corresponds to the percentage of "Pleasant". The central part corresponds to the percentage "Neither unpleasant, nor pleasant" and the part just below to the percentage "Unpleasant". The last part (inferior) of the bars corresponds to the percentage "Very unpleasant".

The sample D7R obtained the greatest percentage of pleasantness of the flavor "very pleasant", according to Figure 94.

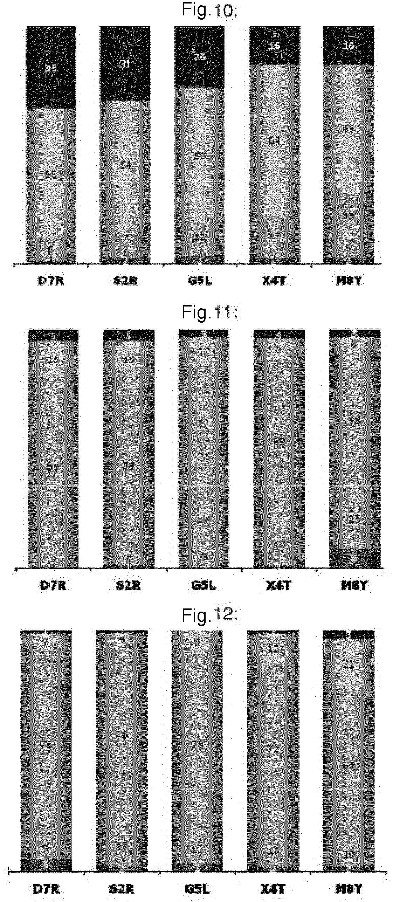

According to Figure 10, regarding the ease of application, the sample with the greatest index of "very easy to apply" was D7R, followed by S2R. It can be noticed that the superior part of the bars corresponds to the percentage of "Very easy to apply", while the part just below corresponds to the percentage of "Easy to apply". The central part corresponds to the percentage "neither difficult, nor easy" and the part just below to the percentage "Hard to apply". The last part (inferior) of the bars corresponds to the percentage "Very hard to apply".

The sliding of D7R sample (Figure 11) was considered ideal in relation to the rest by the consumers, being the product M8Y considered the worst in this issue. The superior part of the bars corresponds to the percentage of "A lot more than I like", while the part just below corresponds to the percentage of "more than I like". The central part corresponds to the percentage "ideal, the way I like it" and the part just below to the percentage "Less than I like". The last part (inferior) of the bars corresponds to the percentage "A lot less than I like".

According to Figure 12, the ideal texture for the stick was preferred in product D7R. The superior part of the bars corresponds to the percentage of "A lot harder/firmer than I like", while the part just below corresponds to the percentage of "harder/firmer than I like". The central part corresponds to the percentage "ideal, the way I like it" and the part just below to the percentage "mushier/softer than I like". The last part (inferior) of the bars corresponds to the percentage "a lot mushier/softer than I like".

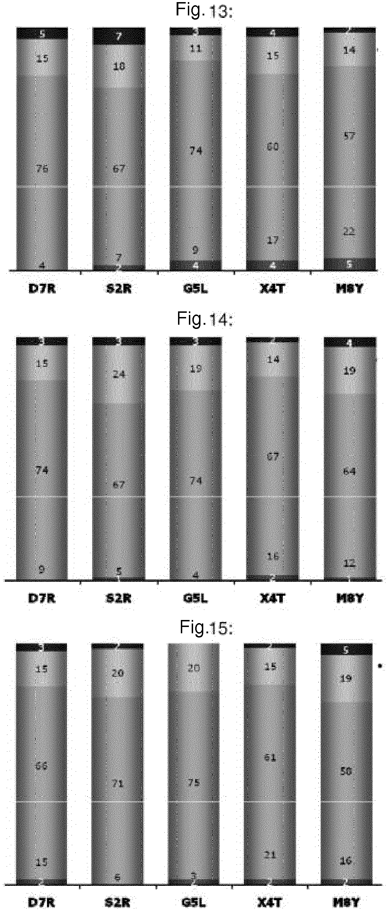

The ideal emollience was obtained, according to Figure 13, by the sample D7R, followed by G5L. The superior part of the bars corresponds to the percentage of "Much more emollient/soft than I like", while the part just below corresponds to the percentage of "more emollient/soft than I like". The central part corresponds to the percentage "ideal/emollient, the way I like it" and the part just below to the percentage "less emollient/soft than I like". The last part (inferior) of the bars corresponds to the percentage "Much less emollient/soft than I like".

The amount discharged for the samples D7R and G5L was ideal for 74% of the interviewees according to Figure 14. The superior part of the bars corresponds to the percentage of "Much bigger than the ideal", while the part just below corresponds to the percentage of "bigger than the ideal". The central part corresponds to the percentage "ideal, as I like it" and the part just below to the percentage "smaller than the ideal". The last part (inferior) of the bars corresponds to the percentage "Much smaller than the ideal".

The coverage (amount of color deposited on the lips), according to Figure 15, was considered ideal for 75% of the interviewees in the case of sample G5L. The superior part of the bars corresponds to the percentage of "Much more coverage than I like", while the part just below corresponds to the percentage of "more coverage than I like". The central part corresponds to the percentage "ideal/ presents coverage the way I like it" and the part just below to the percentage "less coverage than I like". The last part (inferior) of the bars corresponds to the percentage "Much less coverage than I like".

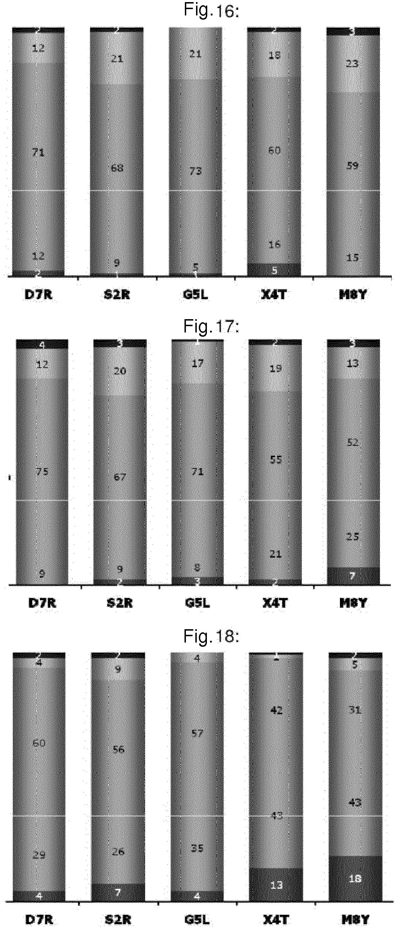

According to Figure 16, the thickness of the film on the lips left by the sample G5L was considered ideal by 73% of the participants in the survey. The superior part of the bars corresponds to the percentage of "Much thicker than I like", while the part just below corresponds to the percentage of "thicker than I like". The central part corresponds to the percentage "ideal/ presents a thickness of the film the way I like it" and the part just below to the percentage "Less thick than I like". The last part (inferior) of the bars corresponds to the percentage "Much less thick than I like".

Figure 17 discloses that the creaminess of the film was considered ideal by 75% of the participants for a sample D7R, followed by sample G5L (71%). In this Figure, the superior part of the bars corresponds to the percentage of "Much more creamy than I like", while the part just below corresponds to the percentage of "More creamy than I like". The central part corresponds to the percentage "ideal/ presents a creaminess of the film the way I like it" and the part just below to the percentage "Less creamy than I like". The last part (inferior) of the bars corresponds to the percentage "Much less creamy than I like".

The shine was considered ideal for 60% of the interviewees in the case of sample D7R, followed by 57% for a sample G5L according to Figure 18. The superior part of the bars corresponds to the percentage of "Much more shine than I like", while the part just below corresponds to the percentage of "More shine than I like". The central part corresponds to the percentage "ideal/ provides the shine the way I like it" and the part just below to the percentage "Less shine than I like". The last part (inferior) of the bars corresponds to the percentage "Much less shine than I like".

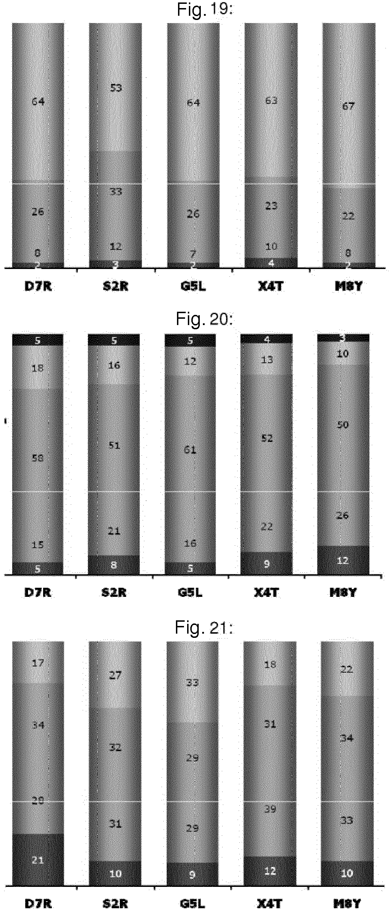

The product considered to be the one that less runny by 67% of the interviewees was M8Y, according to Figure 19. The superior part of the bars corresponds to the percentage of "Doesn't run", while the part just below corresponds to the percentage of "Runs little". The part just below corresponds to the percentage "Runs" and the last part (inferior) of the bars corresponds to the percentage "Runs too much".

The hydration/softness considered "very superior" for 5% of those interviewed for the products D7R, S2R and G5L, according to Figure 20. The superior part of the bars corresponds to the percentage of "Much more hydrated/soft than usual", while the part just below corresponds to the percentage of "More hydrated/soft than usual". The central part corresponds to the percentage "Hydrated/soft as usual" and the part just below to the percentage "less hydrated/soft than usual". The last part (inferior) of the bars corresponds to the percentage "Much less hydrated/soft than usual".

Figure 21 discloses that the products with bigger duration time are G5L, followed by S2R. The superior part of the bars corresponds to the percentage of "Quite satisfactory", while the part just below corresponds to the percentage of "Satisfactory". The central part corresponds to the percentage of "Neither unsatisfactory, nor satisfactory". The part just below corresponds to the percentage "Unsatisfactory" and the last part (inferior) of the bars corresponds to the percentage "Quite unsatisfactory".

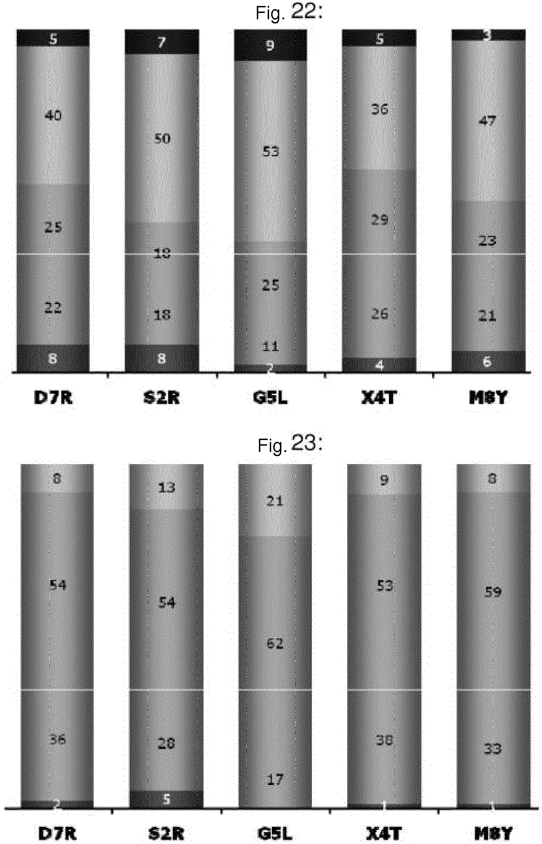

The same result is obtained for the items "Satisfaction with the duration", according to Figure 22, and "Fixation", according to Figure 23. In Figure 22, the superior part of the bars corresponds to the percentage of "Much more hydrated/soft than usual", while the part just below corresponds to the percentage of "More hydrated/soft than usual". The central part corresponds to the percentage "Hydrated/soft as usual" and the part just below to the percentage "less hydrated/soft than usual". The last part (inferior) of the bars corresponds to the percentage "Much less hydrated/soft than usual". In Figure 23, the superior part of the bars corresponds to the percentage of "Has high fixation on the lips", while the part just below corresponds to the percentage of "Has fixation on the lips". The part just below corresponds to the percentage "Has low fixation on the lips" and the last part (inferior) of the bars corresponds to the percentage "Does not fix to the lips".

These three Figures (Figures 21 to 23) are extremely important, since they reveal that:

- 62% of the consumers were satisfied with the duration time of the lipstick of the present invention (G5L). 62% corresponds to the sum of 2-3 h with more than 3 hs;
- 21% of the consumers classidied the lipstick of the present invention (G5L) as a product with high fixation (less than 1/3 of the sample studied);
- 62% of the consumers classified as satisfactory or very satisfactory this duration time (Figure 22); and
- 33% of the consumers perceived more than 3 h of duration of the lipstick of the present invention (Figure 21).
- 83% of the consumers perceived the lipstick of the present invention as being a product that fixes to the lips. (Figure 23)

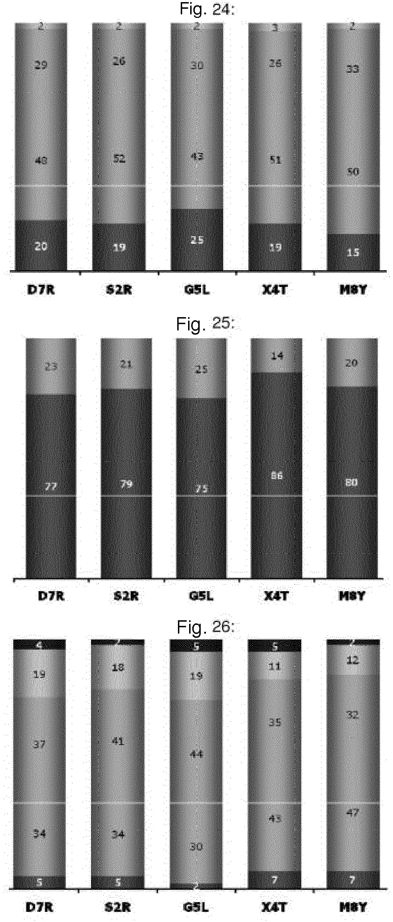

The transfer of the product did not obtain significant differences between the samples (Figure 24). In this Figure, the superior part of the bars corresponds to the percentage of "Does not transfer", while the part just below corresponds to the percentage of "Has low transfer". The part just below corresponds to the percentage "Has average transfer" and the last part (inferior) of the bars corresponds to the percentage "Has high transfer".

Most of the interviewees did not consider the products as something new, however, the sample GL5 presented the biggest number of yes for this question according to Figure 25. The superior part of the bars corresponds to the percentage of "Yes" and the inferior part to the percentage of "No".

The samples G5L and X4T presented more consumers classifying them as "much better that the usual product" according to Figure 26. The superior part of the bars corresponds to the percentage of "Much better than my usual product", while the part just below corresponds to the percentage of "Better than my usual product". The central part corresponds to the percentage "the same as my usual product" and the part just below to the percentage "Worse than my usual product". The last part (inferior) of the bars corresponds to the percentage "Much worse than my usual product".

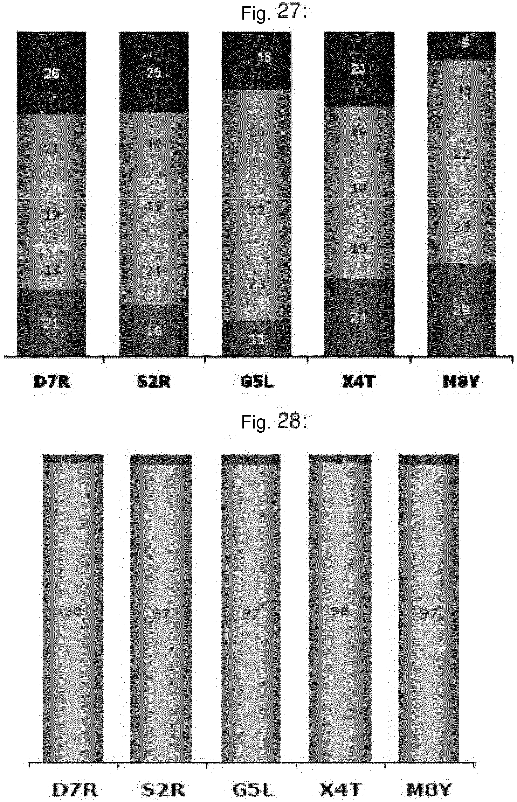

The sample D7R, followed by S2R had had the biggest indexes of preference according to a Figure 27. The superior part of the bars corresponds to "first", while the part just below corresponds to "second". The central part corresponds to "third" and the part just below to "fourth". The last part (inferior) of the bars corresponds to "fifth".

Most of the interviewees did not have any adverse reaction to the products tested according to Figure 28. The superior part of the bars corresponds to "felt" and the inferior part to "did not feel".

From these tests, it can be concluded that the sample M8Y was considered that of worst performance among samples evaluated M8Y comprises a formulation without silica microspheres and emollients, which are present in G5L.

In general, the sample G5L presented a superior performance to the samples X4T and M8Y, despite not being different from X4T in some attributes. In the comparison with D7R and S2R, the sample G5L also presented similar performance and several times better in the attributes evaluated.

Continuing with the market research, the tests below consist of phrases presented to the interviewees, which must evaluate each product tested and say how much she agrees or disagrees with respect to if the product tested possesses these characteristics. The superior part of the bars corresponds to "completely agree", while the part just below corresponds to "partially agree". The central part corresponds to "not agree nor disagree" and the part just below "partially disagree". The last part (inferior) of the bars corresponds to "completely disagree".

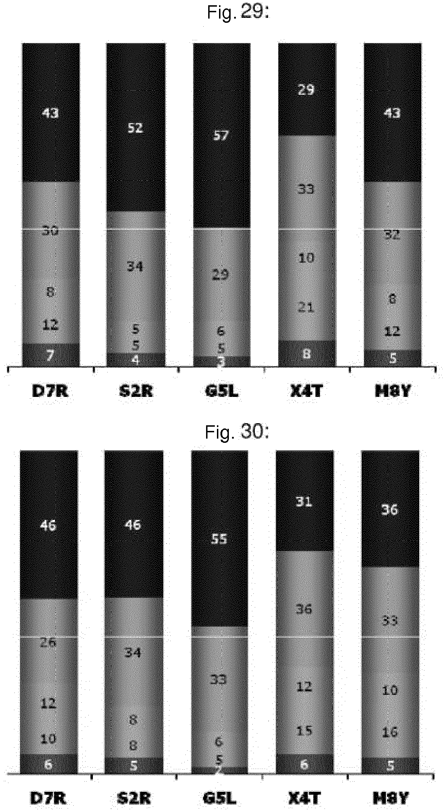

With respect to the sensation of hydrated/soft lips even without the lipstick, 57% of the interviews agreed completely with this affirmation, with respect to product G5L (Figure 29).

In relation to the delivery of high coverage at the first swipe, 55% of the interviews agreed completely with this affirmation with respect to product G5L (Figure 30).

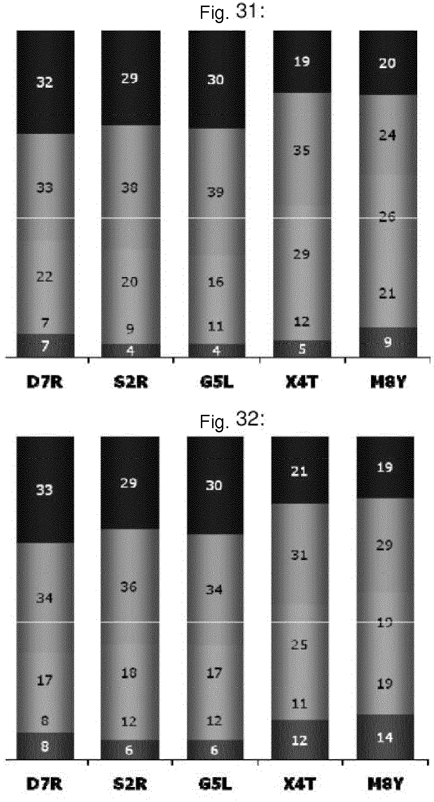

The sensation of protected lips was felt totally by 32% of the interviews for D7R and 30% for G5L (Figure 31).

The Figure 32 discloses that 33% of the interviewees felt their lips less dry for product D7R followed by 30% of the interviewees for G5L.

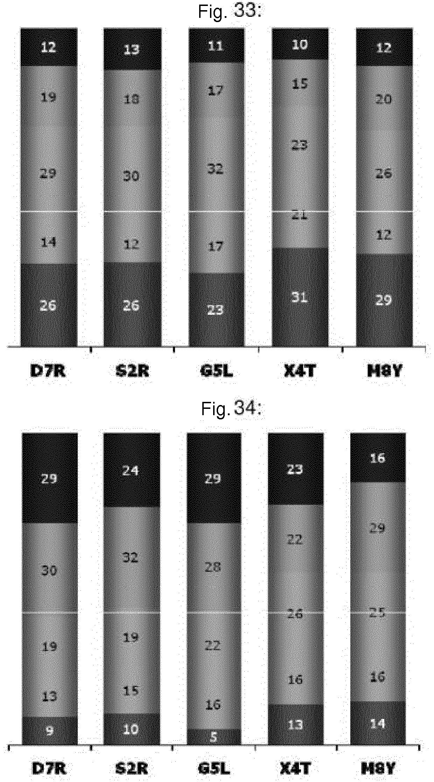

With respect to the sensation that the volume of the lips increased, 13% of the interviews agreed completely with the affirmation for S2R (Figure 33).

For 29% of the interviewees, the use of the samples D7R and G5L reduced the dryness of the lips, and whitish skin which peeled (Figure 34).

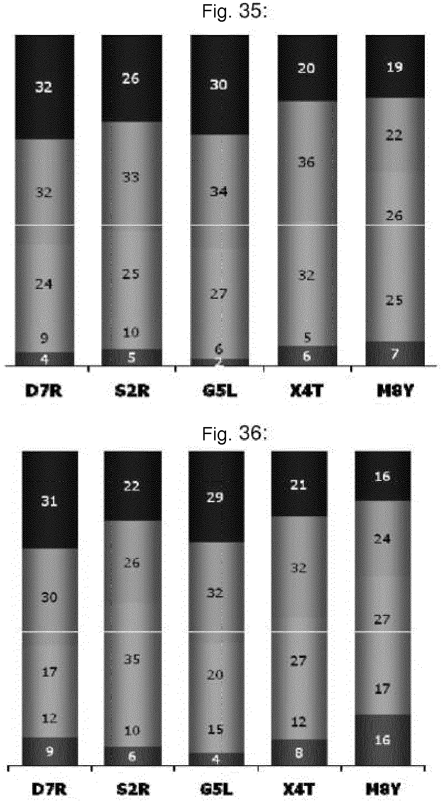

Figure 35 discloses that 32% of the interviewees had a pleasurable sensation in the mouth with product D7R, followed by 30% of the interviewees for G5L.

Figure 36 discloses that 31 % of the interviewees had a sensation of hydrated lips for 24h with product D7R, followed by 29% of the interviewees for G5L.

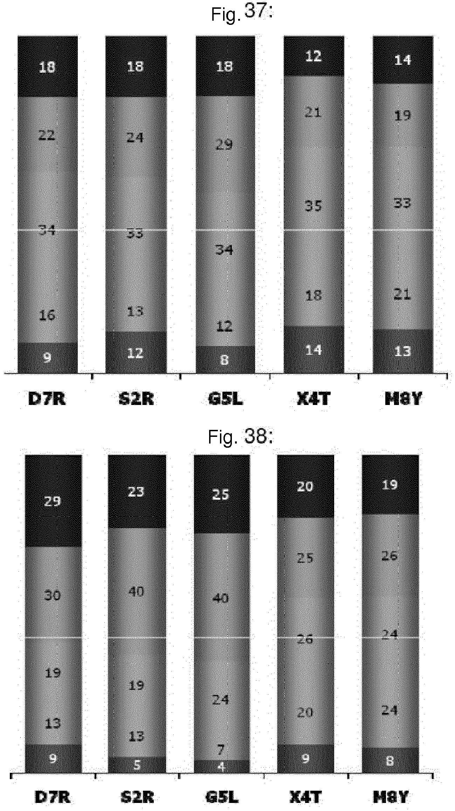

Products D7R, SR2 and G5L had 18% of the interviewees fully agreeing with the statement that these products improve the definition of the lips (Figure 37)

Figure 38 discloses that 29% of the interviewees had a sensation of improvement in the general appearance of the lips with the product D7R, followed by 25% of the interviewees for G5L.

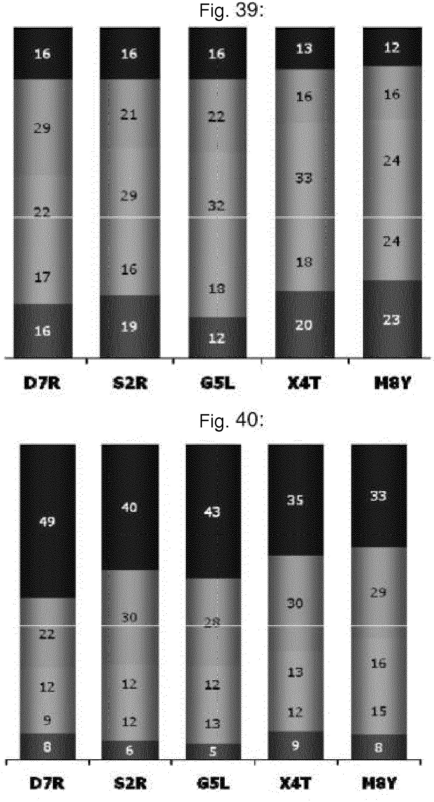

The products D7R, SR2 and G5L had 16% of the interviewees fully agreeing with a statement that these products give a sensation of comfort (Figure 39).

Figure 40 discloses that 49% of the interviewees thought that the product D7R does not make the lips slimy / sticky, followed by 43% of the interviewees for G5L.

The products D7R and G5L had 28% of the interviewees fully agreeing with a statement that these products provide intense color in the first application (Figure 41).

Figure 42 discloses that 22% of the interviewees had a sensation that the product D7R disguises as thin lines of the lips, followed by 19% of the interviewees for G5L.

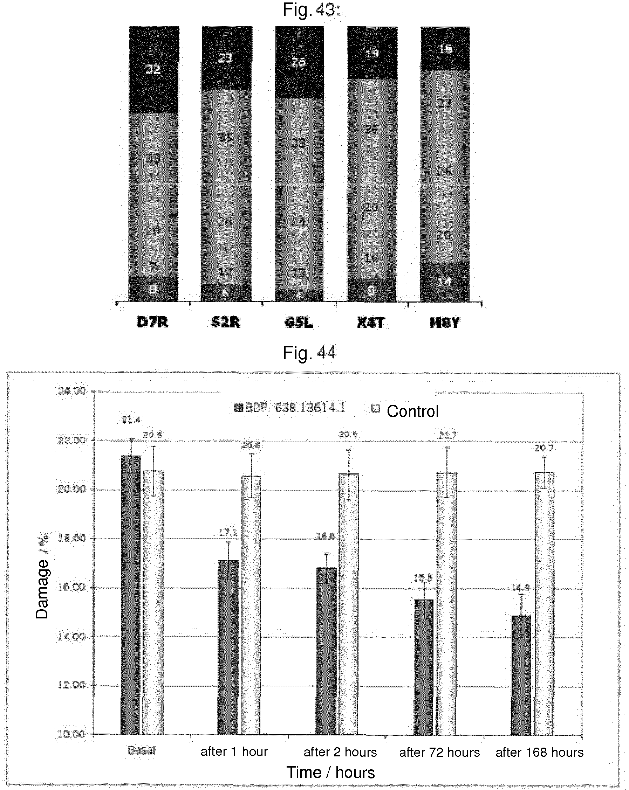

Figure 43 discloses that 32% of the interviewees had a sensation that the product D7R provides softer lips, followed by 26% of the interviewees for G5L.

• *Test 2: Labial restoration*

Periods of low temperatures and humidity, generally in winter, can cause severe damage to the skin, such as alteration to the sensitivity, cracked lips and an increase in dermatitis. As well as the seasonal effect, other factors contribute to the occurrence of skin conditions, such as excessive exposure to solar radiation or frequent use of surfactant products.

The dryness of the skin is one of the most common skin conditions in human beings. This disorder is characterized by superficial roughness and peeling, and is generally associated with sensations of a loss of shine, irritation, redness and pain.

In many cases the use of cosmetic products can minimize or eliminate these skin conditions. These cosmetics act maintaining the stratum corneal hydrated by two biophysical mechanisms: the first is related to lipidic substances based mainly on fatty acids altering the regions of the skin, that is, creating a barrier against the loss of water. The second mechanism is described by a natural humidifying factor, which consists of a mixture of hydrosoluble compounds with a high hygroscopic character. These compounds act against the dehydration maintaining the skin soft and humid.

The repairing activity in cosmetic products is due mainly to the quality and amount of emollients and humectants present, especially with respect to the hydrophilic capacity of these products.

The evaluation of the repairing effect of cosmetic products can be undertaken through a series of methods of study which are internationally recognized and widely applied.

The use of micro cameras permits the microscopic evaluation of the skin, with magnifications of 10x to 700x, obtaining information which can vary from texture and topography to color aspects.

However, this technique, when used by itself, only permits the subjective comparisons between regions or states of the skin before and after treatment.

The use of image analysis software permits, however, the obtainment of quantitative information through the images collected.

For that, the main step that determines the applicability and the success of the technique is the definition of the parameters of evaluation which shall be used, even before the obtainment of the digital images.

Once defined the parameter of evaluation it becomes necessary to obtain standardized and representative images.

Finally, the use of a calculation algorithm permits the obtainment of information which is used generally in a comparative manner between study groups and/or between a standard/control.

The present study had as its objective the evaluation of the efficacy of the cosmetic of the present invention applied on the lips as to the restorative and standardization of the texture attributes in relation to the labial skin.

The evaluations employed to test the efficacy of the products were done beforehand and in the unfolding of the study, after 1, 2, 72 hours (3 days) and 168 hours (7 days). The study was conducted on 2 groups of 11 volunteers each:

• product: group of volunteers that used the product of the present invention during the study.
• control: group of volunteers which remained without the use of any labial products during the study.

The evaluation of the efficacy was undertaken in volunteers who presented dry lips and with lines, through analysis of the images acquired with a CCD micro camera.

The evaluation of the efficacy of the product in the restoration and standardization of the texture of the labial skin was undertaken with the peeling parameter. The result obtained is reported as Percentage of Damage or % Damage. The values of the % Damage for each volunteer of each group evaluated are described in Table 3. In Figure 44 are presented the average values of the Percentage of Damage (% Damage).

Table 3: Values (%) of initial of Damage and after treatment

| Sample Product of the Present invention | | | | | |
|---|---|---|---|---|---|
| Volunteer code | Base | 1h | 2h | 72h | 168h |
| NT701-01 | 20.88 | 16.70 | 16.69 | 15.76 | 15.53 |
| NT701-02 | 21.18 | 16.17 | 15.77 | 13.98 | 13.60 |
| NT701-03 | 21.51 | 16.31 | 16.11 | 14.40 | 12.84 |
| NT701-04 | 20.38 | 16.48 | 16.43 | 15.55 | 15.38 |
| NT701-05 | 20.56 | 16.81 | 16.59 | 15.41 | 15.47 |
| NT701-06 | 21.39 | 16.63 | 16.45 | 15.71 | 15.06 |

(continued)

| Sample Product of the Present invention | | | | | |
|---|---|---|---|---|---|
| Volunteer code | Base | 1h | 2h | 72h | 168h |
| NT701-07 | 21.11 | 17.79 | 17.37 | 15.64 | 14.81 |
| NT701-08 | 22.67 | 18.49 | 17.66 | 16.23 | 15.01 |
| NT701-09 | 21.45 | 17.84 | 17.16 | 16.60 | 15.45 |
| NT701-15 | 21.51 | 17.15 | 17.15 | 15.82 | 14.91 |
| NT701-32 | 22.41 | 17.63 | 17.32 | 15.60 | 15.54 |
| NT701-10 | 21.34 | 20.64 | 20.77 | 21.62 | 21.06 |
| NT701-11 | 21.05 | 20.68 | 20.62 | 20.87 | 20.41 |
| NT701-12 | 21.49 | 21.65 | 21.59 | 21.61 | 21.11 |
| NT701-13 | 21.67 | 21.07 | 21.05 | 21.67 | 21.81 |
| NT701-14 | 19.52 | 19.50 | 19.81 | 20.51 | 20.07 |
| NT701-16 | 20.36 | 20.22 | 20.19 | 18.76 | 20.74 |
| NT701-24 | 18.52 | 18.58 | 18.05 | 19.07 | 19.49 |
| NT701-26 | 21.66 | 21.08 | 21.50 | 21.25 | 21.13 |
| NT701-27 | 21.52 | 21.42 | 21.56 | 21.63 | 21.01 |
| NT701-29 | 20.79 | 20.60 | 20.69 | 20.25 | 20.75 |
| NT701-30 | 20.49 | 20.95 | 21.00 | 20.64 | 20.46 |

To evaluate the significance of the results obtained, a comparative statistical analysis was undertaken between the values of % Damage of the product groups (group of volunteers that used the product) and control (group of volunteers which remained without the use of any labial products during the study period), using the Student's test-t, bimodal, non-paired, with an interval of 95% confidence (Dcontrol, ti vs. Dproduct, ti, i = 0, 1, 2, 72, 168 h). The results are summarized on Table 4, below:

Table 4: Comparison of % Damage between Product and Control in the times evaluated

| Base | 1h | 2h | 72h | 168h |
|---|---|---|---|---|
| 0,1149 | P<0,0001 | P<0,0001 | P<0,0001 | P<0,0001 |

According to the results, there was no statistically significant difference between the groups of volunteers at the initial time, that is, base (P > 0,05). This indicates that the study was undertaken with homogenous groups of volunteers in relation to the intensity if the initial labial % Damage.

There was a significant reduction in the % Damage in the group of volunteers that used the product according to present invention when compared to the group of volunteers that did not use any products (control group) on the lips (P < 0,05) in all of the times analyzed, that is, after 1, 2, 72 and 168 hours of the application.

A statistical analysis was undertaken of the average results of the % Damage in 1 h, 2h, 72h (3 days) and 168h (7 days) with respect to the base, using the test t-Student, bimodal, paired, considering an interval of confidence of 95% (Dti vs. Dt0; i = 0, 2, 72, 168h). The complete results are summarized on Table 5, below:

Table 5: Summarized statistic. Comparison between t0 and ti, where i = 1, 2, 72 and 168 h. Value of P.

| | Product Nature Code BDP: 638. 13614.1 | Control |
|---|---|---|
| After 1 hour | P<0,0001 | 0,1169 |
| After 2 hours | P<0,0001 | 0,2172 |
| After 72 hours | P<0,0001 | 0,8152 |

(continued)

|  | Product Nature Code BDP: 638. 13614.1 | Control |
|---|---|---|
| After 168 hours | P<0,0001 | 0,8303 |

When compared to the base, the product presented a reduction of the % Damage which was statistically significant after 1, 2, 72 and 168 hours of use. A positive evolution was also observed in the effect of the product in relation to the reduction of labial damage, including the minimization of the dryness of the lips and an increase in the standardization of the labial skin, evidenced by a reduction in peeling.

For the group of volunteers that did not use any products on the lips, control group, it was observed that the labial damage remained unaltered over time.

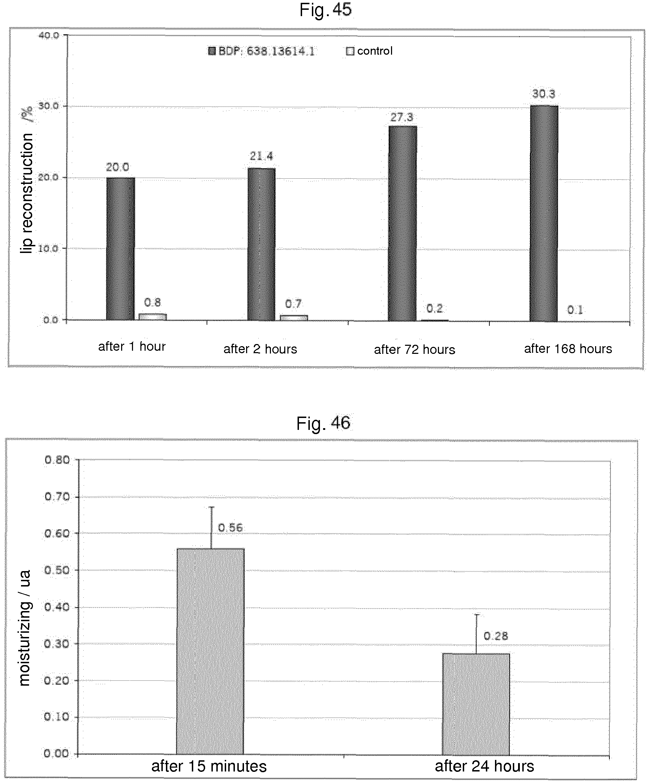

The percentage of labial restoration (%RL) with respect to the initial state, conferred by the use of the product of the present invention was calculated using Equation 1, whose results are presented in Figure 45.

$$\%RL = \frac{100\,(D_{to} - D_{ti})}{D_{to}} \qquad \text{Equation 1}$$

wherein: $D_{to}$ is the base level of damage (t0) and $D_{ti}$ is the level of damage after use for 1, 2; 72 or 168 hours.

Therefore, it was possible to evidence that the use of the product of the present invention conferred a reduction in the Percentage of Damage (% Damage) which was statistically significant with respect to control after 1, 2, 72 hours (3 days) and 168 hours (7 days) ($P < 0,05$; á = 0,05). This indicates that the use of the product restores and standardizes the texture of the labial skin. Furthermore, there was an average reduction of the % Damage, which was statistically significant, of approximately 30%, after continuous use of the product in question for 7 consecutive days when compared to the initial state of the labial skin.

• *Test 3: Evaluation of the hydration of the skin by FTIR-ATR*

The hydration of the skin has been considered a topic of great interest, both in the medical field and in the chemical-cosmetic field. Skin considered dry, in general, presents a rough appearance, without softness and flexibility. These signs are caused by the state of hydration of the skin.

The control of hydration is extremely complex because it does not deal with a single factor. The state of hydration is a result of the action of numerous substances, such as, for example, hygroscopic molecules, free amino acids known as the natural hydration factor (NMF) and of extra-cellular substances, like lipids of the lamellar layers responsible for the cohesion of the stratum corneal.

The moisturizing cosmetic formulas can have one of two functions: avoid the loss of water forming a barrier against superficial evaporation (occlusive), or they can hydrate the stratum corneum by the water contained in the product applied or through absorption of the water in the atmosphere (moisture).

Among other methods used to quantify the hydration of the skin and the hygroscopic properties of cosmetic preparations, we have: analysis of the electrical capacitance of the skin (Colorneometry), loss of transepidermal water (TEWL) and spectroscopic analyses (FTIR-ATR).

The FTIR-ATR ("Attenuated Total Reflectance Fourier Transform Infrared Spectroscopy") is a non destructive method, versatile and effective for the analysis of hydration. Measurements of FTIR-ATR have the following advantages:

• Optical Constant: Depth of penetration can be controlled by the choice of the crystal and angle of incidence;
• Characterization of films: Different layers can be obtained using the subtraction of spectrums;
• Micro-analyses: High sensitivity in micro-regions.

According to studies undertaken by Brancaleon and collaborators (Brancaleon L. et. al. Regional variation and penetration profile of molecular components in human stratum Corneum investigated with in vivo attenuated total reflection-Fourier transform infrared spectroscopy. J. Invest. DermatoL 2001; 116:380-386), the hydration of the skin, in the region of the forearm, can be obtained through the ratio between the intensity of the peaks observed in the spectrums of FTIR-ATR in 2920 cm-1, with reference to the aliphatic chains of lipids of the skin (asymmetrical stretching CH2), and in 3300 cm-1, with reference to symmetrical stretching O-H of water.

However, the product applied also presents peaks of absorption in 2920 cm-1, which makes it difficult to use.

Wichrowski (Wichrowski K, Sore G and Khaiat A. Use of Infrared Spectroscopy for In Vivo Measurement of the Stratum Corneum Moisturization After Application of Cosmetic Preparations. Int. J. Cosmt. ScL 1995, 17:1-11) used i his work the relation between the absorbencies at 1650 cm-1 (Amida I) and 1550 cm-1 (Amida II) for the determination of the hydric content of the skin.

The band Amida I is a result of stretching C=O (70 to 85%) and C-N (10 to 20%). The frequency of the absorption is between 1600 to 1700 cm-1 and the exact position is determined by the protein chain and by the hydrogen bonds (Krimm S, Bandekar J. Vibrational spectroscopy and conformation of peptides, polypeptides, and proteins. Adv Protein Chem. 1986; 38:181-364; Arrondo JL, Muga A, Castresana J, Goni FM. Quantitative studies of the structure of proteins in solution by Fourier-transform infrared spectroscopy. Prog. Biophys. Mo.1 Biol. 1993; 59(1):23-56).

The band Amida II occurs between 1510 and 1580 cm-1 and is more complex than Amida I, being derived, mainly, form the angular deformation in the bonding plane N-H (40 to 50%). The remainder of the energy comes from the stretching C-N (18-40 %) and C-C (about 10%) (Krimm, 1986; Arrondo, 1993).

In previous work, Potts (Potts RO, Guzek DB, Harris RR and McKie J AND. A Noninvasive, In Vivo Technique to Quantitatively Measure Water Concentration of Stratum Corneum Using Attenuated Total-Reflectance Infrared Spectroscopy. Arch. DermatoL Res. 1985, 277:489-95), preferred the use of the water band in 2100 cm-1 to the use of the Amida I/Amida II relation, due to problems in the interpretation of the results.

In this study, the results were analyzed from the relations between the areas of the bands in 1650 cm-1 and 1550 cm-1 with reference to the spectrum obtained (Exsymol. Cutaneous Analysis by Infrared Spectroscopy, Hydration Measurent, D.S.H.0 - T4, Monaco, 1993.).

The present study had as its objective evaluating the level of hydration of the skin after application of the product of the present invention on the skin of the volar forearm region of 20 volunteers. The measurements of the hydration of the skin were done by the FTIR-ATR technique. The experimental procedure was undertaken according to what is below.

In each volunteer two sites of 2,5 x 4,0 cm in the left or right forearm were marked. One site remained without the application of any products, as Control. On the other site 20,0 mg of sample was applied, spreading it using a finger cot. The correlation between site and sample was randomized.

To guarantee that the product would stay in contact with the skin during the entire study, an adhesive tape was placed on the sites with product and control, for protection.

The volunteers were instructed not to remove the protections and not to wet the sites during the study.

The volunteers remained in a acclimatized room (22 $\pm$ 2°C and 50 $\pm$ 5% UR) during 20 minutes before each measurement.

The spectrums of the skin in its initial condition were obtained, before the application of the product, 15 minutes and 24 hours after the application of the product.

Before each reading, the protection of the site was carefully removed and the product was carefully removed using cotton in hydrophilic balls, dry, three sessions of constant compression, in the same direction. The same procedure was simulated for the control site.

After the first reading the product was reapplied and the protection remade.

The spectrums were obtained by the main researcher, positioning the forearm of the volunteer over the FTIR-ATR cell, undertaking 20 cumulative sweeps on each.

At 3300 cm-1 the occur bands for the absorption of stretching O-H of water with influence from stretching N-H of proteins (band Amida-A; Krimm, 1986; Arrondo, 1993) and, therefore, only the absorptions in 1650 cm-1 and 1550 cm-1 were considered in the calculations of the absorption ratios, seeking to evidence the hydration effect of the skin.

From the FTIR-ATR spectrums, the areas of the peaks were calculated with reference to the absorptions in 1650 and 1550 cm-1 (Exsymol, 1993).

From these values, the ratio (**R**) was calculated between the areas **A1650/A1550** and the respective values for hydration as a function of the initial state and the control, according to the equations 1 and 2.

$$\Delta h_{ti} = R_{ti} - R_{to} \qquad \text{Equation 1}$$

wherein: ti = value in time i= 1, 2 or 3 h; t0 = initial or base value

$$H_{ti} = \Delta h_{produto,ti} - \Delta h_{controle,ti} \quad \text{Equation 2}$$

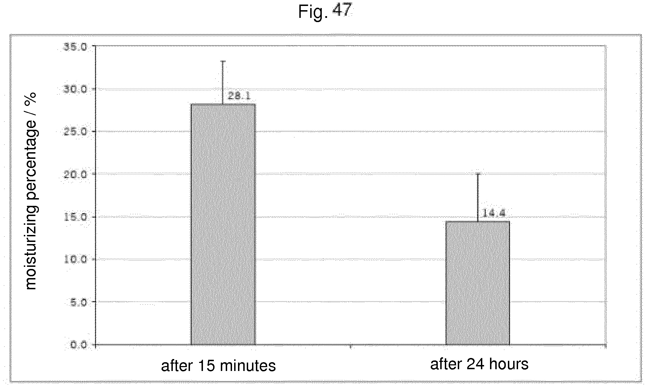

The Figures 46 and 47 illustrate the results calculated for hydration with respect to control, H and Percentage of Hydration, %H respectively.

The values obtained for the ratio A1650/A1550 were compared for a sample and control using the Student's test t, bimodal, paired, with interval of Student confidence of comparing the initial time (t0) with the other available times (t1 and t2). The summarized results are described in Table 6, below.

Table 6: Summarized results of the comparison to vs ti. Values P. alpha = 0,05

| Comparison Groups | Product of the invention | Control |
|---|---|---|
| t0 vs t1 | 0,0001 | 0,2648 |
| t0 vs t2 | 0,0222 | 0,6706 |

The sample presented a significant increase (P<0,05) of the ratio A1650/A1550 after 15 minutes and 24 hours from application, with respect to the base state. In the control site there was no significant difference (P>0,05) after 15 minutes and 24 hours when compared to the base value.

To evaluate the efficacy of the sample with respect to control, the data calculated was analyzed statistically comparing the values of $\Delta h$ of sample vs. Control for each time, using the test t-Student, bimodal, paired, with an interval of confidence of 95%. The summarized results are described in Table 7, below.

Table 7: Comparison of the values of $\Delta h$ of sample vs. Control for each evaluated time. Values of P, alpha = 0,05

| | $\Delta h$, 15 min | $\Delta h$, 24h |
|---|---|---|
| Product vs. control | P<0,0001 | 0,0016 |

The hydration of the skin conferred by the product of the invention presented a statistically significant difference (P > 0,05) after 15 minutes and 24 hours from application. This indicates that the product maintained the skin hydrated for a period of up to 24 hours.

Therefore, the application of sample of the product of the invention on the skin in the region of the forearm, conferred statistically significant hydration, when compared to the control (skin without application of any products) 15 minutes and 24 hours after the application of the product. This indicated that the product of the present invention hydrated the skin.

The application of the product on the skin in the region of the forearm maintained the skin hydrated for up to 24 hours after the application.

## Claims

1. Cosmetic composition for the lips, **characterized by** comprising spherical micro particles of silicon dioxide, emollients and gel, as well as cosmetically acceptable excipients.

2. Cosmetic composition for the lips according to claim 1, **characterized by** comprising at least one of the components selected from the group consisting of ceramides, cocoa butter, vegetable lanolin, Bis-diglycerylpolyacyladipate-2, lycopene, Vitamin E, green coffee extract, pigments, film forming agents, sunscreens, ingredients with a high refractive index.

3. Cosmetic composition for the lips according to claim 2, **characterized in that** the ceramides are passion fruit ceramides.

4. Cosmetic composition for the lips according to claim 1 or 2, **characterized by** being in the form of a lipstick or lip gloss.

5. Cosmetic composition for the lips according to any one of claims 1 to 4, **characterized by** comprising:

| Component name | Concentration (%) |
|---|---|
| Lipstick base | 40,0 - 60,0 |
| Maleated castor oil | 0,5 - 2,5 |
| Theobrom cacao seed butter | 0,1 - 3 |
| Octyldodecanol | 1,0 - 10,0 |
| Dyes | 2,0 - 20,0 |

(continued)

| Component name | Concentration (%) |
| --- | --- |
| Fractionated-hydrogenated vegetable fat | 0,3 - 3,0 |
| Castor oil | 0,5 - 25,0 |
| Dicaprylyl ether | 4,0 - 19,0 |
| Silica microspheres | 0,5 - 2,5 |
| 3-[2-(ethylhexyl)oxyl]-1,2-propanediol | 0,2 - 0,7 |
| Flavoring | 0,5 - 2,5 |
| Passion fruit ceramides | 0,2- 1,0 |

6. Cosmetic composition for the lips according to claim 5, **characterized by** the fact that the lipstick base comprises:

| Component name | Concentration (%) |
| --- | --- |
| Isocetyl stearoyl stearate | 5.0 - 25.0 |
| Diisostearyl malate | 1.0 - 5.0 |
| Dicaprylyl ether | 4.0 - 19.0 |
| Glyceryl abietate | 0.5 - 3.0 |
| Castor oil | 0.5 - 25.0 |
| Fractionated-hydrogenated vegetable fat | 0.3 - 3.0 |
| Lycopene | 0.02 - 0.15 |
| Ceresin P. | 1.0 - 4.0 |
| Microcrystalline wax | 1.0 - 4.0 |
| Bis-diglyceryl 2-polyacyladipate | 0.5 - 3.0 |
| Dipentaeritrityl polyhydroxystearate | 0.5 - 3.0 |
| Candellila wax | 1.0 - 10.0 |
| Organic carnauba wax | 1.0 - 10.0 |

7. Cosmetic composition for the lips according to any one of claims 1 a 6, **characterized by** providing a synergic effect of sliding and delivery of high coverage in a single application, supplying the renewal and hydration of the lips.

Fig. 1

Fig. 2

Fig. 3

Fig. 4:

| D7R | S2R | G5L | X4T | M8Y |

Fig. 5:

| D7R | S2R | G5L | X4T | M8Y |

Fig. 6:

| D7R | S2R | G5L | X4T | M8Y |

Fig. 7:

| D7R | S2R | G5L | X4T | M8Y |

Fig. 8:

| D7R | S2R | G5L | X4T | M8Y |

Fig. 9:

| D7R | S2R | G5L | X4T | M8Y |

Fig.10:

Fig.11:

Fig.12:

Fig. 13:

D7R    S2R    G5L    X4T    M8Y

Fig. 14:

D7R    S2R    G5L    X4T    M8Y

Fig. 15:

D7R    S2R    G5L    X4T    M8Y

Fig.16:

Fig.17:

Fig.18:

Fig. 19:

Fig. 20:

Fig. 21:

Fig. 22:

| D7R | S2R | G5L | X4T | M8Y |

Fig. 23:

| D7R | S2R | G5L | X4T | M8Y |

Fig. 24:

Fig. 25:

Fig. 26:

Fig. 27:

| D7R | S2R | G5L | X4T | M8Y |

Fig. 28:

| D7R | S2R | G5L | X4T | M8Y |

Fig. 29:

Fig. 30:

Fig. 31:

| D7R | S2R | G5L | X4T | M8Y |

Fig. 32:

| D7R | S2R | G5L | X4T | M8Y |

Fig. 33:

| D7R | S2R | G5L | X4T | M8Y |
|-----|-----|-----|-----|-----|

Fig. 34:

| D7R | S2R | G5L | X4T | M8Y |
|-----|-----|-----|-----|-----|

Fig. 35:

Fig. 36:

Fig. 37:

Fig. 38:

Fig. 39:

Fig. 40:

Fig. 41:

| D7R | S2R | G5L | X4T | M8Y |
|-----|-----|-----|-----|-----|

Fig. 42:

| D7R | S2R | G5L | X4T | M8Y |
|-----|-----|-----|-----|-----|

Fig. 43:

Fig. 44

Fig. 45

Fig. 46

Fig. 47

## INTERNATIONAL SEARCH REPORT

| International application No |
|---|
| PCT/BR2011/000306 |

**A. CLASSIFICATION OF SUBJECT MATTER**
INV. A61Q1/04     A61Q1/06     A61K8/25     A61K8/02     A61K8/68
ADD.

According to International Patent Classification (IPC) or to both national classification and IPC

**B. FIELDS SEARCHED**

Minimum documentation searched (classification system followed by classification symbols)
A61K   A61Q

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| X | US 2002/197222 A1 (FISHMAN YORAM [US]) 26 December 2002 (2002-12-26) | 1-4 |
| Y | paragraph [0021] tables 1-4 ----- | 1-7 |
| X | US 5 034 216 A (BARONE SALVATORE J [US] ET AL) 23 July 1991 (1991-07-23) cited in the application | 1-4 |
| Y | example 4 column 2, line 38 - line 52 ----- | 1-7 |
| X | EP 1 439 816 B1 (DEGUSSA [DE] EVONIK DEGUSSA GMBH [DE]) 15 October 2008 (2008-10-15) cited in the application | 1,2,4 |
| Y | paragraph [0053]; table 2 paragraph [0060] ----- | 1-7 |
|  | -/-- |  |

[X] Further documents are listed in the continuation of Box C.      [X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 22 June 2012 | 03/07/2012 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Cismaru, L |

Form PCT/ISA/210 (second sheet) (April 2005)

## INTERNATIONAL SEARCH REPORT

International application No

PCT/BR2011/000306

C(Continuation). DOCUMENTS CONSIDERED TO BE RELEVANT

| Category* | Citation of document, with indication, where appropriate, of the relevant passages | Relevant to claim No. |
|---|---|---|
| X | EP 1 671 614 A1 (OREAL [FR]) 21 June 2006 (2006-06-21) | 1-4 |
| Y | paragraph [0057] paragraph [0062] examples 1,3 ----- | 1-7 |
| Y | FR 2 915 375 A1 (OREAL [FR]) 31 October 2008 (2008-10-31) example 1 claims 1-12 ----- | 1-7 |

Form PCT/ISA/210 (continuation of second sheet) (April 2005)

## INTERNATIONAL SEARCH REPORT
Information on patent family members

International application No

PCT/BR2011/000306

| Patent document cited in search report | | Publication date | Patent family member(s) | | Publication date |
|---|---|---|---|---|---|
| US 2002197222 | A1 | 26-12-2002 | NONE | | |
| US 5034216 | A | 23-07-1991 | NONE | | |
| EP 1439816 | B1 | 15-10-2008 | AT | 411086 T | 15-10-2008 |
| | | | CA | 2465615 A1 | 08-05-2003 |
| | | | CN | 1633277 A | 29-06-2005 |
| | | | DE | 10153077 A1 | 22-05-2003 |
| | | | EP | 1439816 A1 | 28-07-2004 |
| | | | JP | 4102757 B2 | 18-06-2008 |
| | | | JP | 2005508973 A | 07-04-2005 |
| | | | WO | 03037287 A1 | 08-05-2003 |
| EP 1671614 | A1 | 21-06-2006 | AT | 469632 T | 15-06-2010 |
| | | | EP | 1671614 A1 | 21-06-2006 |
| | | | ES | 2346231 T3 | 13-10-2010 |
| | | | FR | 2879439 A1 | 23-06-2006 |
| FR 2915375 | A1 | 31-10-2008 | NONE | | |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6548075 B
- WO 2009022306 A
- EP 1439816 A
- WO 2008022836 A
- US 5034216 A

**Non-patent literature cited in the description**

- **BRANCALEON L.** Regional variation and penetration profile of molecular components in human stratum Corneum investigated with in vivo attenuated total reflection-Fourier transform infrared spectroscopy. *J. Invest. DermatoL,* 2001, vol. 116, 380-386
- **WICHROWSKI K ; SORE G ; KHAIAT A.** Use of Infrared Spectroscopy for In Vivo Measurement of the Stratum Corneum Moisturization After Application of Cosmetic Preparations. *Int. J. Cosmt. ScL,* 1995, vol. 17, 1-11
- **KRIMM S ; BANDEKAR J.** Vibrational spectroscopy and conformation of peptides, polypeptides, and proteins. *Adv Protein Chem.,* 1986, vol. 38, 181-364
- **ARRONDO JL ; MUGA A ; CASTRESANA J ; GONI FM.** Quantitative studies of the structure of proteins in solution by Fourier-transform infrared spectroscopy. *Prog. Biophys. Mo.1 Biol.,* 1993, vol. 59 (1), 23-56
- **POTTS RO ; GUZEK DB ; HARRIS RR ; MCKIE J ; A NONINVASIVE.** In Vivo Technique to Quantitatively Measure Water Concentration of Stratum Corneum Using Attenuated Total-Reflectance Infrared Spectroscopy. *Arch. DermatoL Res.,* 1985, vol. 277, 489-95
- Exsymol. Cutaneous Analysis. Hydration Measurent, D.S.H.0 - T4, Monaco. Infrared Spectroscopy, 1993